# EUROPEAN PATENT APPLICATION

(11) **EP 3 988 131 A1**
(43) Date of publication of application: **27.04.2022**
(21) Application number: 21203782.4
(22) Date of filing: 20.10.2021
(51) Int. Cl.: A61L 2/18, A61L 2/20, A61L 9/14

(54) **SANITISATION TUNNEL**

(30) Priority: 22.10.2020 IT 202000024916
(71) Applicant: Modulpoint S.r.l., 37040 Arcole (VR) (IT)
(72) Inventor: RANCAN, Stefano, 36070 TRISSINO (VI) (IT)
(74) Representative: Marchioro, Paolo

(57) **Abstract**

The present invention relates to a sanitisation tunnel (10), comprising a crossing corridor (11) in turn comprising:
- an entrance passage (12) with a first mobile barrier (13) for the one-way passage of a single person;
- a hand disinfection zone (14) with a sanitisation device (15) for a user's hands;
- a body disinfection zone (16) with nebulisation means (17) configured to nebulise a disinfectant substance inside the crossing corridor (11);
- a suction platform (18) configured to suction dust from a user's feet, and the nebulised disinfectant substance not deposited on the user;
- thermal detection means (19) configured to automatically measure a user's body temperature;
- an exit passage (20) with a second mobile barrier (21) for the one-way passage of a single person.

## Description

The invention relates to a sanitisation tunnel.

The spread of the Covid19 virus makes the need to provide everyone with evermore tools to limit contagion increasingly pressing, and it has been found that prevention and disinfection are the most suitable weapons for limiting such a spread.

Nowadays, in the context of access to public and private facilities such as hospitals, retirement homes, schools, museums, ministries, public offices, as well as all places of public utility and all private companies, disinfection operations are left to impromptu arrangements at the building entrance, with dispensers for a hand sanitiser and a hand-held thermo-scanner which must be handled either by an attendant or by the person who must enter the building.

It has therefore become very difficult to ensure a proper disinfection of people entering an environment from the outside.

Another problem with the disinfection and control systems known to date is that they are designed to disinfect people and not the objects which people carry with them; this leads to the persistence of a very high risk of contagion due to the contact of sanitised hands with an object, such as a bag or an item of clothing, which is brought into an environment from the outside.

The task of the present invention is to develop a sanitisation tunnel capable of overcoming the aforementioned drawbacks and limitations of the known sanitisation systems.

In particular, an object of the invention is to develop a sanitisation tunnel capable of carrying out a sanitisation even of items carried or worn by a user. Another object of the invention is to develop a compact, i.e., space-saving, sanitisation tunnel which can also be easily installed in a passage of an existing building.

A further object of the invention is to develop a sanitisation tunnel which is easily sanitised in the event of a person crossing it who is suspected of being infected.

Further, an object of the invention is to develop a sanitisation tunnel capable of operating very quickly.

The above-mentioned task and objects are achieved by a sanitisation tunnel according to claim 1.

Further features of the sanitisation tunnel according to claim 1 are described in the dependent claims.

The aforesaid task and objects, together with the advantages which will be mentioned hereinafter, are indicated by the description of an embodiment of the invention, which is given by way of non-limiting example with reference to the attached drawings, where:

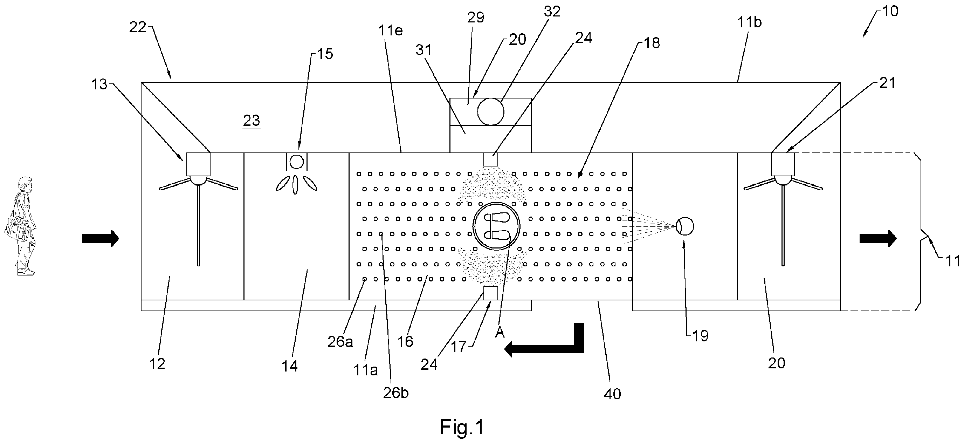

- figure 1 depicts a schematic plan view of a sanitisation tunnel according to the invention;

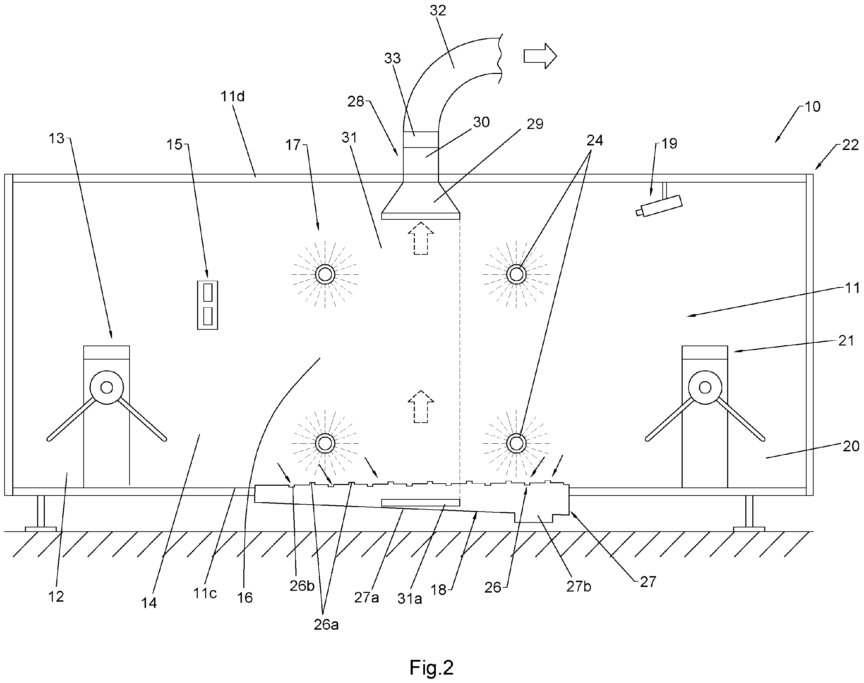

- figure 2 depicts a schematic side view of a sanitisation tunnel according to the invention.

With reference to the cited figures, a sanitisation tunnel according to the invention is indicated as a whole by number **10**.

The sanitisation tunnel **10** comprises a crossing corridor **11**, which in turn comprises:
- an entrance passage **12** with a first mobile barrier **13** for the one-way passage of a single user;
- a hand disinfection zone **14** with a sanitisation device **15** for the hands of a user;
- a body disinfection zone **16** with nebulisation means **17** configured to nebulise a disinfectant substance inside the crossing corridor **11**;
- a suction platform **18** configured to suction dust from the feet of a user, and of the nebulised disinfectant substance not deposited on the user;
- thermal detection means **19** configured to automatically measure a user's body temperature;
- an exit passage **20** with a second mobile barrier **21** for the one-way passage of a single person.

In the present non-limiting embodiment of the invention, the crossing corridor **11** is defined inside a containment structure **22** comprising two side walls, a first side wall **11a** and a second side wall **11b**, a floor **11c** and a ceiling **11**d**.** The containment structure **22** also comprises a technical compartment **23**. Such a technical compartment **23** extends next to the crossing corridor **11**. The technical compartment **23** is separated from the crossing corridor **11** by an intermediate wall **11e.**

The technical compartment **23** is configured to contain the apparatuses for the operation of the means operating inside the crossing corridor **11** and the devices for the control and management of such means.

Therefore, the crossing corridor **11** is defined between a first side wall **11a,** the intermediate wall **11e,** the floor **11c** and the ceiling **11d**.

In the embodiment of the sanitisation tunnel **10** according to the invention described herein, by way of non-limiting example of the invention itself, the first mobile barrier 13 for the one-way passage of a single user comprises a first turnstile.

Alternatively, the first mobile barrier **13** can comprise a revolving or sliding or roto-translating door, as well as other mobile passage control devices, to be understood as being of a known type.

The first mobile barrier **13** is configured to open and close automatically, i.e., without the manual intervention of a user.

The hand disinfection zone **14** is preferably defined downstream of the first mobile barrier **13** and before the body disinfection zone **16**, with respect to an advancement direction from the first mobile barrier **13** to the second mobile barrier **21**.

Those embodiments in which the hand disinfection zone **14** precedes the first mobile barrier **13** and in which the hand disinfection zone **14** at least partially coincides with the body disinfection zone **16** are to be understood as being comprised in the invention.

The hand sanitisation device **15** comprises a pressure or automatic dispenser, or another similar and equivalent device.

The body disinfection zone **16** is preferably, but not exclusively, defined above the suction platform **18**.

The body disinfection zone **16** is located substantially centrally to the crossing corridor **11**.

The nebulisation means **17** comprise, for example, at least one nebulisation nozzle **24** for each of the two opposite walls of the crossing corridor **11**.

In particular, in the present embodiment, the nebulisation means **17** comprise four nebulisation nozzles **24** fixed to the first side wall **11a** and four opposite nebulisation nozzles **24** on the intermediate wall **11e.**

It is to be understood that the nebulisation nozzles **24** can also be in a different number and in different positions depending on technical requirements.

It is also to be understood that one or more nebulisation nozzles can be set up on the ceiling **11d.**

The nebulisation means **17** obviously also comprise one or more corresponding nebulisation apparatuses, each comprising a reservoir of disinfectant substance and means for pumping the disinfectant substance, and connecting ducts between such apparatuses and the corresponding nebulisation nozzles **24**.

The nebulisation apparatuses, not shown for simplicity and intended to be of a known type, are located in the technical compartment **23**.

The suction platform **18** comprises:
- a walkway perforated plate **26**;
- a conveying basin **27,** defined below the perforated plate **26** and configured to collect dust and nebulised substance descending through the holes of the perforated plate **26**;
- extraction means **28** configured to suction air from the conveying basin **27** and expel it from the containment structure **22**.

For example, the perforated plate **26** consists of a perforated flat sheet made of metal material.

For example, such a perforated plate **26** is made of AISI 304 stainless steel.

By way of non-limiting example of the invention, the perforated plate **26** has a first series of holes **26a** for the suction of air and dust, and a second series of holes **26b** for the discharge of liquids which deposit above the perforated plate **26** itself.

Again for example, the perforated plate **26** can comprise a first series of holes **26a** with a diameter of 6 mm, with a density of 280 holes per square metre. Still for example, the perforated plate **26** can comprise a second series of holes **26b** with a diameter of 6 mm, with a density of 50 holes per square metre.

In particular, the first series of holes **26a,** for the suction of air, has along its edge a series of reliefs, or a single annular relief, facing upwards with respect to a normal use arrangement.

Such reliefs, or such a single annular relief, are obtained for example by a punching or shearing operation by which the same first series of holes **26a** is made.

Therefore, such a first series of holes **26a** is also configured to prevent a user walking on the perforated plate **26** from slipping.

The second series of holes **26b** is obtained, for example, by a punching or shearing operation carried out in the opposite direction to the punching or shearing operation by which the first set of holes **26a** is made.

Such a second series of holes **26b** does not have any upward relief on the edge of the holes, but instead has reliefs or a single annular relief, facing downwards, to facilitate the descent of any surrounding liquid.

The holes of the perforated plate **26** are preferably configured to achieve a passage velocity for air which is comprised between 0.4 m/s and 0.5 m/s per hole.

By way of example, and certainly not limiting the invention, the perforated plate 26 can have a width of 110 cm and a length comprised between 200 cm and 400 cm.

Such a perforated plate 26 has anti-slip reliefs, for example as described above, in order to possess anti-slip properties.

Furthermore, such reliefs cooperate in conveying any liquids towards the holes of the perforated plate **26**.

The conveying basin **27** comprises a sloping bottom **27a** for conveying the collected liquids towards a discharge opening **27b**.

The conveying basin **27** extends below the entire extension of the perforated plate **26,** in order to allow the suction of air and liquids from all the holes of the perforated plate **26** itself.

The conveying basin **27** can also be made of metal material, for example AISI 304 stainless steel.

Those embodiments in which the perforated plate **26** is made of other non-metallic materials, such as a plastic material, or a glass material, or a wood material, or another technically equivalent material, are also to be understood as comprised in the invention.

Similarly, those embodiments in which the conveying basin **27** is made of other non-metallic materials, such as a plastic material, or a glass material, or a wood material, or another equivalent material, are also to be understood as comprised in the invention.

The conveying basin **27** is preferably between 5 and 7 cm deep.

In the embodiment of the invention described herein as a non-limiting example of the invention itself, the extraction means **28** comprise:
- a suction hood **29**;
- a suction device **30**;
- a suction duct **31**, configured to connect the inside of the conveying basin **27**, by means of a suction mouth **31a,** with the suction hood **29**;
- an outlet flue **32**.

The suction hood **29** preferably comprises a static filter; still preferably, but not exclusively, such a static filter is a microbial filter, made of filter board.

Such a static filter is for example positioned between the suction hood **29** and the suction device **30**.

The static filter is to be understood as removable.

For example, the suction device **30** comprises a spiral suction unit.

In particular, such a suction unit is of the spiral centrifugal type, with a power of 2.2 KW, configured to achieve a pressure of 145/208 Kgf/m3 and a flow-rate of 22/63; the average output speed is comprised between 8 m/s and 9 m/s.

The extraction means **28** can advantageously also comprise an ionising filter **33**.

For example, such an ionising filter **28** comprises a tubular section within which a plurality of ionising candles are arranged.

Such ionising candles are understood to be for example candle capacitors, for example four, mounted in a special container positioned downstream of the suction device **30**.

Such ionising candles can be between 2 and 6 in number, proportionate to the size of the suction platform **18**.

The outlet flue **32** comprises a pipe, for example a stainless steel pipe.

Such an outlet flue **32** can have a mesh insect filter configured to prevent insects from entering the outlet flue **32** from the outside.

The thermal detection means **19** can comprise, for example, a thermo-scanner fixed to the ceiling **11d** of the crossing corridor **11**, or to a wall of choice between the first side wall **11a** and the intermediate wall **11e**.

The thermal detection means **19** are to be understood as being also others, of a type known per se.

The exit passage **20** is located at the end of the crossing corridor **11**.

The second mobile barrier **21** for the one-way passage of a single user is to be understood as being one of the types described above for the first mobile barrier **13**.

In the zone between the first mobile barrier **13** and the second mobile barrier **21**, the crossing corridor **11** comprises a safety side door **40**, configured to allow the emergency exit of a user, for example if said user has been detected by the thermal detection means **19** as having a body temperature greater than a predetermined value, for example greater than or equal to 37.5°C.

In the present embodiment, the safety side door **40** is defined on the first side wall **11a,** before the second mobile barrier **21**.

The safety side door **40** is preferably defined at, or downstream of, the body disinfection zone **16**.

A sanitisation process which can be carried out with the sanitisation tunnel **10** according to the invention is described below.

An initial operating step of such a process includes that, as a user enters through the first mobile barrier **13**, the suction platform **18** is activated, which removes dust and any viruses from the soles of the user's shoes, the soles of shoes being considered one of the main vehicles for spreading the virus. Thus, the transit through the first mobile barrier **13** results in the turning on of the extraction means **28**.

Before being released to the outside environment, the suctioned air is ionised through the ionising filter **33**, if present.

After passing through the first mobile barrier **13**, the user disinfects his hands with the special disinfectant liquid using the hand sanitisation device **15**.

When the user enters the body disinfection zone **16**, he is asked to position himself on the suction platform **18** at a stop area **A**, indicated for example by the outline of two soles of the feet, on which the user must position himself for the correct detection of the body temperature by the thermal detection means **19**.

The body temperature measurement occurs automatically when the user is at the correct distance from the thermal detection means 19**.**

If the detected body temperature is less than or equal to a predefined limit temperature, e.g., 37.5°C, the disinfectant is nebulised.

If the detected body temperature is greater than the predetermined limit temperature, the second mobile barrier **21** is blocked and the exit through the safety side door **40** is enabled.

The crossing of the second mobile barrier **21** by the user identified as being at risk of carrying contagion is therefore prevented and at the same time the crossing corridor **11** is cleared without the "presumably contagious" user having to retrace his steps with the risk of meeting other users waiting to cross the same sanitisation tunnel **10**.

However, the nebulisation means **17** are operated in order to disinfect the crossing corridor **11** as well.

The nebulisation step lasts for example 10 seconds, but it is understood as being programmable for different durations depending on the needs and particular requirements of the installation.

The user is completely covered by the nebulised substance, comprising, of course, his clothes, objects carried, and any luggage.

The disinfectant substance comprises a solution based on hydrogen peroxide, which is capable of acting on clothes and all objects which the person carries and which are brought in from outside.

The extraction means **28** remain operational for at least the entire duration of the nebulisation.

At the end of the nebulisation step, and on detecting a body temperature less than or equal to the predetermined limit temperature, the second mobile barrier **21** opens and the user can exit the sanitisation tunnel **10** and enter the place at whose passage the sanitisation tunnel **10** has been arranged.

The duration of the suction step, for a single user, is for example fixed at 2 minutes from the exit of the user; differently, for a plurality of users passing one after the other, the suction step proceeds continuously.

The sanitisation tunnel **10** is configured to be accessible and therefore usable by handicapped persons and personnel with wheelchairs.

It is also possible to consider different sanitisation procedures depending on the type of environment at whose entrance the sanitisation tunnel **10** is arranged, subjecting the most varied objects which must be introduced into such an environment to the nebulisation.

A suction platform **18** as described above in its parts and as a whole is also to be understood as an object of the invention.

In particular, such a suction platform **18** comprises:
- a walkway perforated plate **26**;
- a conveying basin **27**, defined below the perforated plate **26** and configured to collect dust and nebulised substance descending through the holes of the perforated plate **26**;
- extraction means **28** configured to suction air from the conveying basin **27** and expel it from the containment structure **22**.

More in particular, the perforated plate **26** has a first series of holes **26a** for the suction of air and dust, and a second series of holes **26b** for the discharge of liquids which deposit above the perforated plate **26** itself.

In particular, the first series of holes **26a**, for the suction of air, has along its edge a series of reliefs, or a single annular relief, facing upwards with respect to a normal use arrangement of the suction platform **18**.

Such reliefs, or such a single annular relief, are obtained for example by a punching or shearing operation by which the same first series of holes **26a** is made.

Therefore, such a first series of holes **26a** is also configured to prevent a user walking on the perforated plate **26** from slipping.

The second series of holes **26b** is obtained, for example, by a punching or shearing operation carried out in the opposite direction to the punching or shearing operation by which the first series of holes **26a** is made, i.e., the holes of the second series of holes **26b** have along their edge a series of reliefs, or a single annular relief, facing downwards with respect to a normal use arrangement of the suction platform **18**.

Such a second series of holes **26b** does not have any upward relief on the edge of the holes, but has reliefs, or a single annular relief, facing downwards, to facilitate the descent of any surrounding liquid.

The extraction means **28** as described above are also to be understood as being part of the assembly of components referred to with the terms 'suction platform' **18**.

Practically, it has been established that the invention achieves the intended task and objects.

In particular, the invention has developed a sanitisation tunnel capable of also sanitising the items carried or worn by a user.

In addition, the invention has developed a compact sanitisation tunnel, i.e., of limited dimensions, which can be easily arranged even at a passage in an existing building; in fact, the containment structure **22** can consist of a housing module which is easily transportable with transport vehicles of a known type, such as vans, lorries and the like.

Furthermore, the invention has developed a sanitisation tunnel which can be easily sanitised in the event of a person suspected of being infected crossing it, by virtue of the safety side door **40** which allows an alternative and equally safe route to be arranged for such a suspected infected user.

Further, the invention has developed a sanitisation tunnel capable of operating very quickly.

Last but not least, the invention has developed a suction platform which can also be used in other applications, by virtue of its non-slip structure and its ability to suction from therein both liquid and aeriform fluids.

The invention thus conceived is susceptible to numerous modifications and variations, all of which are within the scope of the inventive concept; moreover, all the details may be replaced by other technically equivalent elements.

In practice, the components and materials used, as long as they are compatible with the specific use, as well as the dimensions and the contingent shapes can be any one according to requirements and the prior art.

If the features and techniques mentioned in any claim are followed by reference signs, such reference signs are to be intended for the sole purpose of increasing the intelligibility of the claims and, consequently, such reference signs have no limiting effect on the interpretation of each element identified by way of example by these reference signs.

## Claims

1. Sanitisation tunnel (10), comprising a crossing corridor (11) in turn comprising:
- an entrance passage (12) with a first mobile barrier (13) for the one-way passage of a single person;
- a hand disinfection zone (14) with a sanitisation device (15) for a user's hands;
- a body disinfection zone (16) with nebulisation means (17) configured to nebulise a disinfectant substance inside the crossing corridor (11);
- a suction platform (18) configured to suction dust from a user's feet, and the nebulised disinfectant substance not deposited on the user;
- thermal detection means (19) configured to automatically measure a user's body temperature;
- an exit passage (20) with a second mobile barrier (21) for the one-way passage of a single person,
**characterised in that** said suction platform (18) comprises:
- a walkway perforated plate (26);
- a conveying basin (27), defined below said perforated plate (26) and configured to collect dust and nebulised substance descending through the holes of said perforated plate (26);
- extraction means (28) configured to suction air from said conveying basin (27) and expel it from said containment structure (22).

2. Sanitisation tunnel according to claim 1, **characterised in that** said crossing corridor (11) is defined inside a containment structure (22) comprising two side walls, a first side wall (11a) and a second side wall (11b), a floor (11c) and a ceiling (11d).

3. Sanitisation tunnel according to the preceding claim, **characterised in that** said containment structure (22) comprises a technical compartment (23), said technical compartment (23) being separated from the crossing corridor (11) by an intermediate wall (11e).

4. Sanitisation tunnel according to one or more of the preceding claims, **characterised in that** said nebulisation means (17) comprise at least one nebulisation nozzle (24) for each of the two opposite walls of the crossing corridor (11).

5. Sanitisation tunnel according to claim 1, **characterised in that** said perforated plate (26) consists of a perforated flat sheet made of metal material.

6. Sanitisation tunnel according to claims 1 or 5, **characterised in that** said perforated plate (26) has a first series of holes (26a) for the suction of air and dust, and a second series of holes (26b) for the discharge of liquids deposited above the perforated plate (26) itself.

7. Sanitisation tunnel according to one or more of the preceding claims, **characterised in that** said conveying basin (27) comprises a sloping bottom (27a) for conveying the collected liquids towards a discharge opening (27b), said conveying basin (27) extending below the entire extension of the perforated plate (26), in order to allow the suction of air and liquids from all the holes of the perforated plate (26) itself.

8. Sanitisation tunnel according to one or more of the preceding claims, **characterised in that** said extraction means (28) comprise:
- a suction hood (29);
- a suction device (30);
- a suction duct (31), configured to connect the inside of the conveying basin (27), by means of a suction mouth (31a), with said suction hood (29);
- an outlet flue (32).

9. Sanitisation tunnel according to one or more of the preceding claims, **characterised in that,** in a zone between said first mobile barrier (13) and said second mobile barrier (21), the crossing corridor (11) comprises a safety side door (40), configured to allow an emergency exit of a user.
